# EUROPEAN PATENT APPLICATION

(11) **EP 1 852 435 A1**
(43) Date of publication of application: **07.11.2007**
(21) Application number: 06007667.6
(22) Date of filing: 12.04.2006
(51) Int. Cl.: C07D 473/32, A61K 31/52

(54) **A process for the manufacture of famciclovir using phase-transfer catalysts**

(71) Applicant: SOLMAG S.p.A., 26837 Mulazzano LO (IT)
(72) Inventor: Chiodini, Giorgio, 20010 Inveruno (MI) (IT); Rossi, Alessia, 20024 Garbagnate Milanese (MI) (IT)
(74) Representative: Minoja, Fabrizio

(57) **Abstract**

The invention relates to a process for the preparation of Famciclovir, which comprises the preparation of a compound of formula (II) 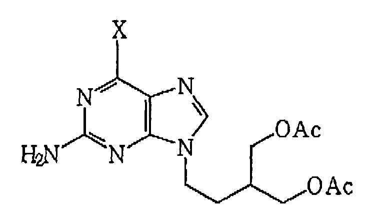 wherein X is a halogen atom
by reaction of a compound of formula (III) 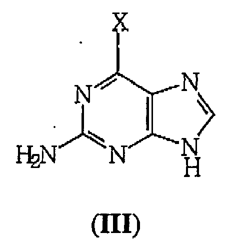 with a compound of formula (VII) 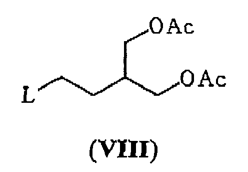 wherein L is a leaving group, in the presence of a catalytic amount of a quaternary ammonium salt,
followed by hydrogenation of compound (II) to Famciclovir.

## Description

### FIELD OF THE INVENTION

The present invention relates to an improved process for the preparation of Famciclovir.

### BACKGROUND OF THE INVENTION

Famciclovir, 2-amino-9-(4-acetoxy-3-acetoxymethylbut-1-yl)purine of formula **(I),** 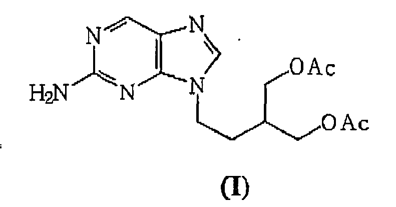 is a known antiviral agent, whose synthesis is disclosed, for example, in EP 182024. The process comprises the hydrogenolysis of a 2-amino-6-halopurine derivative of formula (**II**), 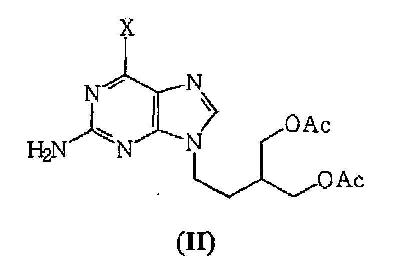 wherein X is chlorine, bromine or iodine,
on Pd/C as catalyst, using methanol containing ammonium formate as the solvent.

EP 0141927 and US 5886215 disclose a process for the preparation of the compound of formula (**II**), which comprises treating a compound of formula **(III)**, 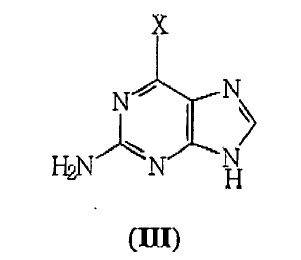 wherein X is as defined above,
with a compound of formula (**IV**) 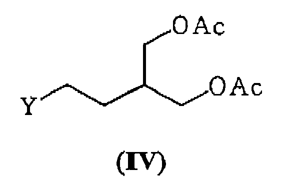 wherein Y is a leaving group, such as Cl, Br or I and Ac is an acetyl group, in an inert organic solvent, preferably dimethylformamide, in the presence of an inorganic base, preferably potassium carbonate. The product is purified through column chromatography on silica gel, whose efficiency is poor from the industrial standpoint.

EP 0141927 discloses the preparation of a compound of formula (**IV**) wherein Y is bromine, by brominating compound (**V**) 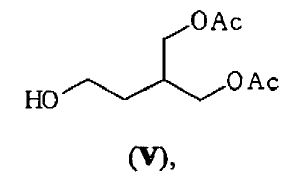 preferably by treatment with an excess of carbon tetrabromide and triphenylphosphine in an aprotic organic solvent, such as dimethylformamide.

This process involves the use of very toxic reagents and leads to formation of by-products that are difficult to waste and requires to purify the bromine derivative by silica gel chromatography; for these reasons, the process is unsuitable for industrial production.

US 5,886,215 discloses the preparation of compound **(IV),** wherein Y is iodine, preferably by treatment of a compound of formula **(VI)** 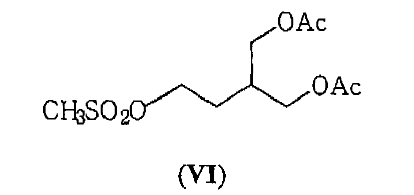 with sodium iodide, in a polar aprotic solvent, such as acetone, followed by silica gel chromatography. Compound (**VI**) is in turn prepared by treating compound (**V**) with methanesulphonyl chloride in dichlorometane, in the presence of triethylamine. This process involves several steps and yields are poor.

US 6,761,767 teaches to directly condense a compound of formula **(III)** with compound **(VI).** This approach reduces the number of steps in respect of US 5,886,215, but the condensation requires prolonged heating, which may be detrimental to the purity of the final product. Moreover, the reaction mixture must be concentrated for the condensation to occur and results in a very thick suspension, which is very difficult to stir; this remarkably lowers the reactivity of the nucleophilic species involved in the reaction. To overcome this problem, it is necessary to add a considerable amount of dimethylformamide, an expensive and toxic solvent.

In view of the above, there is still the need for a process suitable for industrial scale.

### DESCRIPTION OF THE INVENTION

It has now been found that Famciclovir can be conveniently prepared by
a) condensing a compound of formula **(III)** 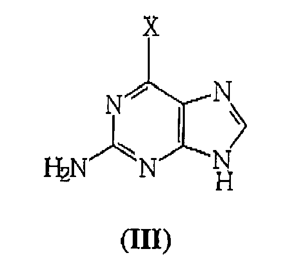 wherein X is a halogen atom
   with a compound of formula **(VII)** 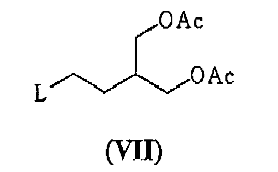 wherein L is a leaving group,
   in the presence of a catalytic amount of a quaternary ammonium salt of formula (**VIII**)

   R₁R₂R₃R₄N⁺Hal⁻ (**VIII**)

   wherein R₁, R₂, R₃ and R₄, which may be the same or different,
   are alkyl or arylalkyl groups and Hal is a halogen atom and in the presence of an inorganic base and an organic solvent to give a compound of formula (**II**) 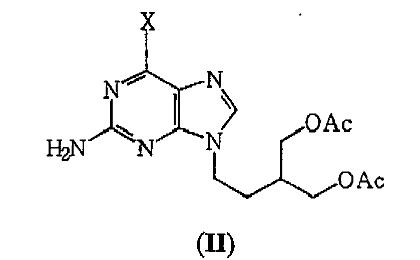
b) hydrogenating compound **(II)** to Famciclovir **(I)**

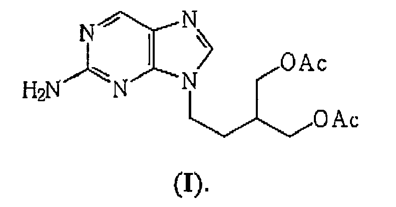

With the aid of the ammonium salt which acts as a phase transfer catalyst, the nucleophilic species involved in the reaction react with the nonionic species in the aprotic polar solvent; the reaction is faster and cleaner and the reaction mixture less thick.

In the compound of formula (**III**), X is preferably selected from chlorine, bromine and iodine; more preferably, X is a chlorine atom.

In the compound of formula (**VII**), the leaving group L is preferably selected from the group consisting of halogens, preferably chlorine, bromine, iodine, more preferably chlorine and bromine, and sulfonyl derivatives, preferably mesylate, triflate, tosylate, nosylate, brosylate, more preferably mesylate and tosylate. According to a preferred embodiment of the invention, compounds (**VII)** are prepared *in situ* from compound (**V**) and are not isolated before the reaction with compounds (**III**).

In the compound of formula (**VIII)**, R₁, R₂, R₃ and R₄ are preferably the same and represent C₂-C₆ alkyl groups. Most preferred compounds of formula (**VIII**) are tetraethylammonium bromide or tetrabutylammonium bromide.

The inorganic base used in step a) is preferably potassium carbonate.

The organic solvents may be aprotic polar or dipolar solvents. Suitable aprotic polar solvents are, for example, acetone, acetonitrile, nitromethane, dimethylformamide, dimethylacetamide, tetramethylurea, N-methylpyrrolidinone, N-methylmorpholine, 1,3-diinethyl-3,4,5,6-tetrahydro-2(1H)-pyrimidinone (DMPU), dimethylsulphoxide (DMSO), tetrahydrothiophen-1,1-dioxide (sulpholane), ethylene glycol diethyl ether and the like.

Preferred aprotic dipolar solvents are amides, dimethylformamide, dimethylacetamide and DMPU being most preferred.

The reaction is carried out at a temperature ranging from 0°C to 150°C, more preferably from 10 to 100°C, and most preferably from 30 to 90°C.

Hydrogenation of compound (**II**) is preferably carried out in methanol in the presence of ammonium formate and Pd/C as catalyst.

The invention will be hereinafter illustrated in more detail by means of the following examples.

### Examples

### Example 1

70.2 ml of triethylamine are added to a stirred solution of 2-acetoxymethyl-4-hydroxybutyl acetate (63.9 g) in toluene; during the addition the temperature is kept at 0°C. The reaction mixture is then cooled to -10°C and 29.5 ml of methanesulphonyl chloride are added drop by drop while keeping the temperature below 0°C. After completion of the addition, the reaction mixture is stirred for a further 1.5 hour, then washed with 350 ml of water and subsequently with 7.02 ml of concentrated HCl. The organic phase is separated from the aqueous phase, which is back-extracted with 140 ml of toluene. The organic fractions are pooled and washed with 170 ml of water. The organic phase is separated and concentrated *in vacuo.* 60 ml of DMF are added and the organic phase is concentrated *in vacuo* again. Finally 600 ml of DMF are added followed by 53.7 g of anhydrous potassium carbonate, 45 g of 6-chloroguanine and 6.5 g of tetraethylammonium bromide. The reaction mixture is stirred for 6 hours at 60-70°C and then is allowed to cool to room temperature. Insoluble materials are filtered off and 810 ml of water are then added to the filtrate, which cause immediate formation of a white precipitate. The slurry is then stirred at 15°C for about one hour. The solid is collected by filtration, washed with water and then dissolved in 365 ml of hot methanol.

1.2 g of charcoal and 2.5 g of diatomaceous earth are added to this solution, which is refluxed for half an hour and then filtered; the filtrate is concentrated *in vacuo* to approximately 250 ml and is finally allowed to crystallize at 10°C. The resulting crystals are filtered and washed with 50 ml of cold methanol.

After drying at 45°C in a drying oven until constant weight, 59.5 g of almost pure (HPLC purity >99%) derivative of formula **(II)** are obtained.

### Example 2

47 g of compound of formula **(II)** and 2.4 g of 10% palladium on charcoal (50% wet) are added under nitrogen to 240 ml of methanol. The mixture is cooled to 5-10°C and then 32 g of ammonium formate are added. The reaction mixture is heated at 50°C for 3 hours. After cooling and filtering the black solid, the filtrate is evaporate to dryness. The residue is partitioned in methylene chloride and water. The organic phase is separated from the aqueous phase and concentrated *in vacuo.* The residue is dissolved in 130 ml of ethyl acetate. 0.5 g of charcoal and 1.0 g of diatomaceous earth are added to this solution, which is refluxed for half an hour. The mixture is then filtered and the filtrate is allowed to crystallize at 15-20°C. The obtained crystals are filtered and washed with 2x25 ml of cold ethyl acetate. After drying at 50°C in a drying oven until constant weight, 33 g of almost pure (HPLC purity >99%) Famciclovir **(I)** are obtained.

## Claims

1. A process for the preparation of Famciclovir (**I**) 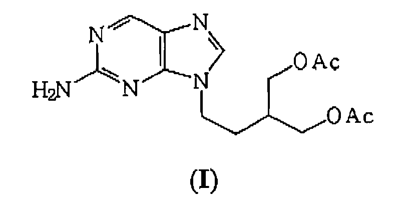 which comprises
a) condensing a compound of formula **(III)** 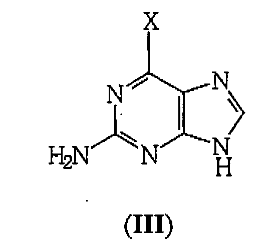 wherein X is a halogen atom
with a compound of formula **(VII)** 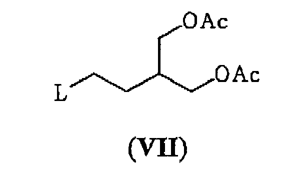 wherein L is a leaving group,
in the presence of a catalytic amount of a quaternary ammonium salt of formula **(VIII)**
R₁R₂R₃R₄N⁺Hal⁻ **(VIII)**
wherein R₁, R₂, R₃ and R₄, which may be the same or different, are alkyl or arylalkyl groups and Hal is an halogen atom
and in the presence of an inorganic base and an aprotic organic solvent to give a compound of formula **(II)** 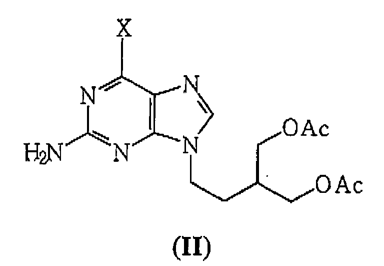
b) hydrogenating compound (**II**) to Famciclovir (**I**).

2. The process of claim 1, wherein X is selected from chlorine, bromine, or iodine.

3. The process of claim 2, wherein X is chlorine.

4. The process of any one of claims 1 to 4, wherein L is selected from the group consisting of chlorine, bromine, iodine, mesylate, triflate, tosylate, nosylate, brosylate.

5. The process of claim 4, wherein L is selected from chlorine, bromine, mesylate and tosylate.

6. The process of any one of claims 1 to 5, wherein R₁, R₂, R₃ and R₄ are the same and represent C₂-C₆ alkyl groups.

7. The process of any one of claims 1 to 6, wherein the aprotic organic solvent is selected from acetone, acetonitrile, nitromethane, dimethylformamide, dimethylacetamide, tetramethylurea, N-methylpyrrolidinone, N-methylmorpholine, 1,3-dimethyl-3,4,5,6-tetrahydro-2(1H)-pyrimidinone, dimethylsulphoxide, tetrahydrothiophen-1,1-dioxide and ethylene glycol diethyl ether.

8. The process of any one of claims 1 to 7, wherein step b) is carried out in methanol in the presence of ammonium formate and Pd/C as catalyst.
